# EUROPEAN PATENT SPECIFICATION

(11) **EP 1 317 551 B1**
(45) Date of publication and mention of the grant of the patent: **28.02.2007**
(21) Application number: 01920329.8
(22) Date of filing: 13.03.2001
(51) Int. Cl.: C12N 15/55, C12N 9/20

(54) **HUMAN PHOSPHOLIPASES AND POLYNUCLEOTIDES ENCODING THE SAME**
MENSCHLICHE PHOSPHOLIPASEN UND DIESE KODIERENDE POLYNUKLEOTIDE
PHOSPHOLIPASES HUMAINES ET POLYNUCLEOTIDES CODANT POUR CELLES-CI

(30) Priority: 13.03.2000 US 188885 P; 15.03.2000 US 189693 P
(43) Date of publication of application: 11.06.2003
(73) Proprietor: Lexicon Genetics Incorporated, The Woodlands, TX 77381-1160 (US)
(72) Inventor: Hu, Yi, The Woodlands, TX 77381 (US); Nepomnichy, Boris, Houston, TX 77909 (US); Donoho, Gregory, Portage MI 49024 (US); Hilbun, Erin, Houston TX 77070 (US); Turner, C. Alexander, Jr., The Woodlands, TX 77381 (US); Abuin, Alejandro, The Woodlands, TX 77382 (US); Friedrich, Glenn, Houston, TX 77004 (US); Zambrowicz, Brian, The Woodlands, TX 77382 (US); Sands, Arthur T., The Woodlands, TX 77382 (US)
(74) Representative: Silveston, Judith
(86) International application number: PCT/US2001/007994
(87) International publication number: WO 2001/068871

(56) References cited:
- DATABASE EBI [Online] Hinxton, UK; Accession number: AQ235271, 29 September 1998 (1998-09-29) XP002183141
- DATABASE EBI [Online] Hinxton, UK; Accession number: B69245, 9 December 1997 (1997-12-09) XP002183142
- DATABASE EBI [Online] Hinxton, UK; Accession number: A27094, 22 August 1996 (1996-08-22) XP002183143
- DATABASE EBI [Online] Hinxton, UK; Accession number: BC006355, 16 April 2001 (2001-04-16) XP002183631
- LEE S B ET AL: "Molecular cloning,splice variants, expression, and purification of phospholipase C-delta4" JOURNAL OF BIOLOGICAL CHEMISTRY, AMERICAN SOCIETY OF BIOLOGICAL CHEMISTS, BALTIMORE, MD, US, vol. 271, no. 1, 5 January 1996 (1996-01-05), pages 25-31, XP002180350 ISSN: 0021-9258
- SONNHAMMER ERIK L L ET AL: "Analysis of protein domain families in Caenorhabditis elegans." GENOMICS, vol. 46, no. 2, 1 December 1997 (1997-12-01), pages 200-216, XP002182511 ISSN: 0888-7543
- FUKAMI ET AL: "Requirement of Phospholipase C-delta 4 for the Zone Pellucida-Induced Acrosome Reaction" SCIENCE, vol. 292, 4 May 2001 (2001-05-04), pages 920-923,

### SEQUENCE LISTING

<110> LEXICON GENETICS INCORPORATED
<120> Novel Human Phospholipases and Polynucleotides Encoding The Same
<130> LEX-0148-PCT
<150> US 60/188,885
   <151> 2000-03-13
<150> US 60/189,693
   <151> 2000-03-15
<160> 20
<170> FastSEQ for Windows Version 4.0
<210> 1
   <211> 720
   <212> DNA
   <213> homo sapiens
<400> 1
   atgctagcag ttaggaaggc caggaggaaa ctcaggatgg ggaccatctg ctcccccaac      60
   cccagcggga caaagacatc atcggaggtc tgcaatgccg actggatggc ctcgctcccc     120
   cctcacctcc acaacctccc cctttccaat ctggcaatcc caggctcaca tgattcattc     180
   agctactggg tggatgaaaa gtccccagtg gggcctgacc aaacccaagc tatcaaacgc     240
   ctcgccagga tctccttggt gaagaagcta atgaagaagt ggtctgtgac tcagaacctg     300
   acatttcgag aacagctgga agctgggatc cgctactttg acctgcgtgt gtcttccaaa     360
   ccaggggatg ccgaccagga gatctacttc atccatgggc tttttggcat caaggtctgg     420
   gatgggctga tggaaattga ctcgtttctt acacagcacc cccaggagat tatcttcctg     480
   gatttcaacc acttctatgc catggatgag acccatcaca aatgcctggt tctgcggatc     540
   caggaggcct ttggaaacaa gctgtgccca gcctgcagtg tggaaagttt gacgctgcga     600
   actctgtggg agaagaactg ccaggtagga gagataaact tccaagagca agaatttaac     660
   tcttctgctt ttcctgtatt gccggctgta aaatcactca atccagggct cttaggctaa     720
<210> 2
   <211> 239
   <212> PRT
   <213> homo sapiens
<400> 2
   Met Leu Ala Val Arg Lys Ala Arg Arg Lys Leu Arg Met Gly Thr Ile
    1               5                  10                  15
   Cys Ser Pro Asn Pro Ser Gly Thr Lys Thr Ser Ser Glu Val Cys Asn
               20                  25                  30
   Ala Asp Trp Met Ala Ser Leu Pro Pro His Leu His Asn Leu Pro Leu
           35                  40                  45
   Ser Asn Leu Ala Ile Pro Gly Ser His Asp Ser Phe Ser Tyr Trp Val
       50                  55                  60
   Asp Glu Lys Ser Pro Val Gly Pro Asp Gln Thr Gln Ala Ile Lys Arg
   65                  70                  75                  80
   Leu Ala Arg Ile Ser Leu Val Lys Lys Leu Met Lys Lys Trp Ser Val
                   85                  90                  95
   Thr Gln Asn Leu Thr Phe Arg Glu Gln Leu Glu Ala Gly Ile Arg Tyr
               100                 105                 110
   Phe Asp Leu Arg Val Ser Ser Lys Pro Gly Asp Ala Asp Gln Glu Ile
           115                 120                 125
   Tyr Phe Ile His Gly Leu Phe Gly Ile Lys Val Trp Asp Gly Leu Met
       130                 135                 140
   Glu Ile Asp Ser Phe Leu Thr Gln His Pro Gln Glu Ile Ile Phe Leu
   145                 150                 155                 160
   Asp Phe Asn His Phe Tyr Ala Met Asp Glu Thr His His Lys Cys Leu
                   165                 170                 175
   Val Leu Arg Ile Gln Glu Ala Phe Gly Asn Lys Leu Cys Pro Ala Cys
               180                 185                 190
   Ser Val Glu Ser Leu Thr Leu Arg Thr Leu Trp Glu Lys Asn Cys Gln
           195                 200                 205
   Val Gly Glu Ile Asn Phe Gln Glu Gln Glu Phe Asn Ser Ser Ala Phe
       210                 215                 220
   Pro Val Leu Pro Ala Val Lys Ser Leu Asn Pro Gly Leu Leu Gly
   225                 230                 235
<210> 3
   <211> 990
   <212> DNA
   <213> homo sapiens
<400> 3
   atgctagcag ttaggaaggc caggaggaaa ctcaggatgg ggaccatctg ctcccccaac     60
   cccagcggga caaagacatc atcggaggtc tgcaatgccg actggatggc ctcgctcccc    120
   cctcacctcc acaacctccc cctttccaat ctggcaatcc caggctcaca tgattcattc    180
   agctactggg tggatgaaaa gtccccagtg gggcctgacc aaacccaagc tatcaaacgc    240
   ctcgccagga tctccttggt gaagaagcta atgaagaagt ggtctgtgac tcagaacctg    300
   acatttcgag aacagctgga agctgggatc cgctactttg acctgcgtgt gtcttccaaa    360
   ccaggggatg ccgaccagga gatctacttc atccatgggc tttttggcat caaggtctgg    420
   gatgggctga tggaaattga ctcgtttctt acacagcacc cccaggagat tatcttcctg    480
   gatttcaacc acttctatgc catggatgag acccatcaca aatgcctggt tctgcggatc    540
   caggaggcct ttggaaacaa gctgtgccca gcctgcagtg tggaaagttt gacgctgcga    600
   actctgtggg agaagaactg ccaggttctt attttctacc actgtccctt ctacaagcag    660
   taccccttcc tgtggccagg aaagaagatt ccagcgccct gggcaaacac cacaagtgtg    720
   cgcaaactaa tcctcttctt ggagaccact ctgagtgagc gggcctcacg gggctccttc    780
   catgtctccc aagcgatcct cacccccaga gtgaagacca ttgcccgggg cttggttggg    840
   ggcctcaaga acacgctggt tcataggaat cttcctgcca tcctggactg ggtgaaaact    900
   cagaagcctg gagccatggg tgtcaacatc atcacatctg acttcgtgga cctggtggac    960
   tttgctgcga ctgtcatcaa agttgaatga                                     990
<210> 4
   <211> 329
   <212> PRT
   <213> homo sapiens
<400> 4
   Met Leu Ala Val Arg Lys Ala Arg Arg Lys Leu Arg Met Gly Thr Ile
   1               5                   10                  15
   Cys Ser Pro Asn Pro Ser Gly Thr Lys Thr Ser Ser Glu Val Cys Asn
               20                  25                  30
   Ala Asp Trp Met Ala Ser Leu Pro Pro His Leu His Asn Leu Pro Leu
           35                  40                  45
   Ser Asn Leu Ala Ile Pro Gly Ser His Asp Ser Phe Ser Tyr Trp Val
           50                      55                      60
   Asp Glu Lys Ser Pro Val Gly Pro Asp Gln Thr Gln Ala Ile Lys Arg
   65                  70                  75                  80
   Leu Ala Arg Ile Ser Leu Val Lys Lys Leu Met Lys Lys Trp Ser Val
                   85                  90                  95
   Thr Gln Asn Leu Thr Phe Arg Glu Gln Leu Glu Ala Gly Ile Arg Tyr
               100                 105                 110
   Phe Asp Leu Arg Val Ser Ser Lys Pro Gly Asp Ala Asp Gln Glu Ile
           115                 120                 125
   Tyr Phe Ile His Gly Leu Phe Gly Ile Lys Val Trp Asp Gly Leu Met
       130                 135                 140
   Glu Ile Asp Ser Phe Leu Thr Gln His Pro Gln Glu Ile Ile Phe Leu
   145                 150                 155                 160
   Asp Phe Asn His Phe Tyr Ala Met Asp Glu Thr His His Lys Cys Leu
                   165                 170                 175
   Val Leu Arg Ile Gln Glu Ala Phe Gly Asn Lys Leu Cys Pro Ala Cys
               180                 185                 190
   Ser Val Glu Ser Leu Thr Leu Arg Thr Leu Trp Glu Lys Asn Cys Gln
           195                 200                 205
   Val Leu Ile Phe Tyr His Cys Pro Phe Tyr Lys Gln Tyr Pro Phe Leu
       210                 215                 220
   Trp Pro Gly Lys Lys Ile Pro Ala Pro Trp Ala Asn Thr Thr Ser Val
   225                 230                 235                 240
   Arg Lys Leu Ile Leu Phe Leu Glu Thr Thr Leu Ser Glu Arg Ala Ser
                   245                 250                 255
   Arg Gly Ser Phe His Val Ser Gln Ala Ile Leu Thr Pro Arg Val Lys
               260                 265                 270
   Thr Ile Ala Arg Gly Leu Val Gly Gly Leu Lys Asn Thr Leu Val His
           275                 280                 285
   Arg Asn Leu Pro Ala Ile Leu Asp Trp Val Lys Thr Gln Lys Pro Gly
       290                 295                 300
   Ala Met Gly Val Asn Ile Ile Thr Ser Asp Phe Val Asp Leu Val Asp
   305                 310                 315                 320
   Phe Ala Ala Thr Val Ile Lys Val Glu
                   325
<210> 5
   <211> 1056
   <212> DNA
   <213> homo sapiens
<400> 5
   atgctagcag ttaggaaggc caggaggaaa ctcaggatgg ggaccatctg ctcccccaac    60
   cccagcggga caaagacatc atcggaggtc tgcaatgccg actggatggc ctcgctcccc   120
   cctcacctcc acaacctccc cctttccaat ctggcaatcc caggctcaca tgattcattc   180
   agctactggg tggatgaaaa gtccccagtg gggcctgacc aaacccaagc tatcaaacgc   240
   ctcgccagga tctccttggt gaagaagcta atgaagaagt ggtctgtgac tcagaacctg   300
   acatttcgag aacagctgga agctgggatc cgctactttg acctgcgtgt gtcttccaaa   360
   ccaggggatg ccgaccagga gatctacttc atccatgggc tttttggcat caaggtctgg   420
   gatgggctga tggaaattga ctcgtttctt acacagcacc cccaggagat tatcttcctg   480
   gatttcaacc acttctatgc catggatgag acccatcaca aatgcctggt tctgcggatc   540
   caggaggcct ttggaaacaa gctgtgccca gcctgcagtg tggaaagttt gacgctgcga   600
   actctgtggg agaagaactg ccaggttctt attttctacc actgtccctt ctacaagcag   660
   taccccttcc tgtggccagg aaagaagatt ccagcgccct gggcaaacac cacaagtgtg   720
   cgcaaactaa tcctcttctt ggagaccact ctgagtgagc gggcctcacg gggctccttc   780
   catgtctccc aagcgatcct cacccccaga gtgaagacca ttgcccgggg cttggttggg   840
   ggcctcaaga acacgctggt tcatagacgg agtctcactc tgtcacccaa actggagtgc   900
   agctcttggc tcaccgcagc ctcaacctcc caggctcagg tgattacccc tcctcacaga   960
   cagggtttca ccatgtttcc caggctgatc tcaaactcct ggattcaagt gatccaccca  1020
   cctcagcccc ccaaagtgcc gggattacag gcatga                            1056
<210> 6
   <211> 351
   <212> PRT
   <213> homo sapiens
<400> 6
   Met Leu Ala Val Arg Lys Ala Arg Arg Lys Leu Arg Met Gly Thr Ile
   1               5                   10                  15
   Cys Ser Pro Asn Pro Ser Gly Thr Lys Thr Ser Ser Glu Val Cys Asn
               20                  25                  30
   Ala Asp Trp Met Ala Ser Leu Pro Pro His Leu His Asn Leu Pro Leu
           35                  40                  45
   Ser Asn Leu Ala Ile Pro Gly Ser His Asp Ser Phe Ser Tyr Trp Val
       50                  55                  60
   Asp Glu Lys Ser Pro Val Gly Pro Asp Gln Thr Gln Ala Ile Lys Arg
   65                  70                  75                  80
   Leu Ala Arg Ile Ser Leu Val Lys Lys Leu Met Lys Lys Trp Ser Val
                   85                  90                  95
   Thr Gln Asn Leu Thr Phe Arg Glu Gln Leu Glu Ala Gly Ile Arg Tyr
               100                 105                 110
   Phe Asp Leu Arg Val Ser Ser Lys Pro Gly Asp Ala Asp Gln Glu Ile
           115                 120                 125
   Tyr Phe Ile His Gly Leu Phe Gly Ile Lys Val Trp Asp Gly Leu Met
       130                 135                 140
   Glu Ile Asp Ser Phe Leu Thr Gln His Pro Gln Glu Ile Ile Phe Leu
   145                 150                 155                 160
   Asp Phe Asn His Phe Tyr Ala Met Asp Glu Thr His His Lys Cys Leu
                   165                 170                 175
   Val Leu Arg Ile Gln Glu Ala Phe Gly Asn Lys Leu Cys Pro Ala Cys
               180                 185                 190
   Ser Val Glu Ser Leu Thr Leu Arg Thr Leu Trp Glu Lys Asn Cys Gln
           195                 200                 205
   Val Leu Ile Phe Tyr His Cys Pro Phe Tyr Lys Gln Tyr Pro Phe Leu
       210                 215                 220
   Trp Pro Gly Lys Lys Ile Pro Ala Pro Trp Ala Asn Thr Thr Ser Val
   225                 230                 235                 240
   Arg Lys Leu Ile Leu Phe Leu Glu Thr Thr Leu Ser Glu Arg Ala Ser
                   245                 250                 255
   Arg Gly Ser Phe His Val Ser Gln Ala Ile Leu Thr Pro Arg Val Lys
               260                 265                 270
   Thr Ile Ala Arg Gly Leu Val Gly Gly Leu Lys Asn Thr Leu Val His
           275                 280                 285
   Arg Arg Ser Leu Thr Leu Ser Pro Lys Leu Glu Cys Ser Ser Trp Leu
       290                 295                 300
   Thr Ala Ala Ser Thr Ser Gln Ala Gln Val Ile Thr Pro Pro His Arg
   305                 310                 315                 320
   Gln Gly Phe Thr Met Phe Pro Arg Leu Ile Ser Asn Ser Trp Ile Gln
                   325                 330                 335
   Val Ile His Pro Pro Gln Pro Pro Lys Val Pro Gly Leu Gln Ala
               340                 345                 350
<210> 7
   <211> 450
   <212> DNA
   <213> homo sapiens
<400> 7
   atgaagaagt ggtctgtgac tcagaacctg acatttcgag aacagctgga agctgggatc      60
   cgctactttg acctgcgtgt gtcttccaaa ccaggggatg ccgaccagga gatctacttc     120
   atccatgggc tttttggcat caaggtctgg gatgggctga tggaaattga ctcgtttctt     180
   acacagcacc cccaggagat tatcttcctg gatttcaacc acttctatgc catggatgag     240
   acccatcaca aatgcctggt tctgcggatc caggaggcct ttggaaacaa gctgtgccca     300
   gcctgcagtg tggaaagttt gacgctgcga actctgtggg agaagaactg ccaggtagga     360
   gagataaact tccaagagca agaatttaac tcttctgctt ttcctgtatt gccggctgta     420
   aaatcactca atccagggct cttaggctaa                                     450
<210> 8
   <211> 149
   <212> PRT
   <213> homo sapiens
<400> 8
   Met Lys Lys Trp Ser Val Thr Gln Asn Leu Thr Phe Arg Glu Gln Leu
   1               5                   10                  15
   Glu Ala Gly Ile Arg Tyr Phe Asp Leu Arg Val Ser Ser Lys Pro Gly
               20                  25                  30
   Asp Ala Asp Gln Glu Ile Tyr Phe Ile His Gly Leu Phe Gly Ile Lys
           35                  40                  45
   Val Trp Asp Gly Leu Met Glu Ile Asp Ser Phe Leu Thr Gln His Pro
       50                  55                  60
   Gln Glu Ile Ile Phe Leu Asp Phe Asn His Phe Tyr Ala Met Asp Glu
   65                  70                  75                  80
   Thr His His Lys Cys Leu Val Leu Arg Ile Gln Glu Ala Phe Gly Asn
                   85                  90                  95
   Lys Leu Cys Pro Ala Cys Ser Val Glu Ser Leu Thr Leu Arg Thr Leu
               100                 105                 110
   Trp Glu Lys Asn Cys Gln Val Gly Glu Ile Asn Phe Gln Glu Gln Glu
           115                 120                 125
   Phe Asn Ser Ser Ala Phe Pro Val Leu Pro Ala Val Lys Ser Leu Asn
       130                 135                 140
   Pro Gly Leu Leu Gly
   145
<210> 9
   <211> 720
   <212> DNA
   <213> homo sapiens
<400> 9
   atgaagaagt ggtctgtgac tcagaacctg acatttcgag aacagctgga agctgggatc      60
   cgctactttg acctgcgtgt gtcttccaaa ccaggggatg ccgaccagga gatctacttc     120
   atccatgggc tttttggcat caaggtctgg gatgggctga tggaaattga ctcgtttctt     180
   acacagcacc cccaggagat tatcttcctg gatttcaacc acttctatgc catggatgag     240
   acccatcaca aatgcctggt tctgcggatc caggaggcct ttggaaacaa gctgtgccca     300
   gcctgcagtg tggaaagttt gacgctgcga actctgtggg agaagaactg ccaggttctt     360
   attttctacc actgtccctt ctacaagcag taccccttcc tgtggccagg aaagaagatt     420
   ccagcgccct gggcaaacac cacaagtgtg cgcaaactaa tcctcttctt ggagaccact     480
   ctgagtgagc gggcctcacg gggctccttc catgtctccc aagcgatcct cacccccaga     540
   gtgaagacca ttgcccgggg cttggttggg ggcctcaaga acacgctggt tcataggaat     600
   cttcctgcca tcctggactg ggtgaaaact cagaagcctg gagccatggg tgtcaacatc     660
   atcacatctg acttcgtgga cctggtggac tttgctgcga ctgtcatcaa agttgaatga     720
<210> 10
   <211> 239
   <212> PRT
   <213> homo sapiens
<400> 10
   Met Lys Lys Trp Ser Val Thr Gln Asn Leu Thr Phe Arg Glu Gln Leu
   1               5                   10                  15
   Glu Ala Gly Ile Arg Tyr Phe Asp Leu Arg Val Ser Ser Lys Pro Gly
               20                  25                  30
   Asp Ala Asp Gln Glu Ile Tyr Phe Ile His Gly Leu Phe Gly Ile Lys
           35                  40                  45
   Val Trp Asp Gly Leu Met Glu Ile Asp Ser Phe Leu Thr Gln His Pro
       50                  55                  60
   Gln Glu Ile Ile Phe Leu Asp Phe Asn His Phe Tyr Ala Met Asp Glu
   65                  70                  75                  80
   Thr His His Lys Cys Leu Val Leu Arg Ile Gln Glu Ala Phe Gly Asn
                   85                  90                  95
   Lys Leu Cys Pro Ala Cys Ser Val Glu Ser Leu Thr Leu Arg Thr Leu
               100                 105                 110
   Trp Glu Lys Asn Cys Gln Val Leu Ile Phe Tyr His Cys Pro Phe Tyr
           115                 120                 125
   Lys Gln Tyr Pro Phe Leu Trp Pro Gly Lys Lys Ile Pro Ala Pro Trp
       130                 135                 140
   Ala Asn Thr Thr Ser Val Arg Lys Leu Ile Leu Phe Leu Glu Thr Thr
   145                 150                 155                 160
   Leu Ser Glu Arg Ala Ser Arg Gly Ser Phe His Val Ser Gln Ala Ile
                   165                 170                 175
   Leu Thr Pro Arg Val Lys Thr Ile Ala Arg Gly Leu Val Gly Gly Leu
               180                 185                 190
   Lys Asn Thr Leu Val His Arg Asn Leu Pro Ala Ile Leu Asp Trp Val
           195                 200                 205
   Lys Thr Gln Lys Pro Gly Ala Met Gly Val Asn Ile Ile Thr Ser Asp
       210                 215                 220
   Phe Val Asp Leu Val Asp Phe Ala Ala Thr Val Ile Lys Val Glu
   225                 230                 235
<210> 11
   <211> 786
   <212> DNA
   <213> homo sapiens
<400> 11
   atgaagaagt ggtctgtgac tcagaacctg acatttcgag aacagctgga agctgggatc 60
   cgctactttg acctgcgtgt gtcttccaaa ccaggggatg ccgaccagga gatctacttc 120
   atccatgggc tttttggcat caaggtctgg gatgggctga tggaaattga ctcgtttctt 180
   acacagcacc cccaggagat tatcttcctg gatttcaacc acttctatgc catggatgag 240
   acccatcaca aatgcctggt tctgcggatc caggaggcct ttggaaacaa gctgtgccca 300
   gcctgcagtg tggaaagttt gacgctgcga actctgtggg agaagaactg ccaggttctt 360
   attttctacc actgtccctt ctacaagcag taccccttcc tgtggccagg aaagaagatt 420
   ccagcgccct gggcaaacac cacaagtgtg cgcaaactaa tcctcttctt ggagaccact 480
   ctgagtgagc gggcctcacg gggctccttc catgtctccc aagcgatcct cacccccaga 540
   gtgaagacca ttgcccgggg cttggttggg ggcctcaaga acacgctggt tcatagacgg 600
   agtctcactc tgtcacccaa actggagtgc agctcttggc tcaccgcagc ctcaacctcc 660
   caggctcagg tgattacccc tcctcacaga cagggtttca ccatgtttcc caggctgatc 720
   tcaaactcct ggattcaagt gatccaccca cctcagcccc ccaaagtgcc gggattacag 780
   gcatga 786
<210> 12
   <211> 261
   <212> PRT
   <213> homo sapiens
<400> 12
   Met Lys Lys Trp Ser Val Thr Gln Asn Leu Thr Phe Arg Glu Gln Leu
    1               5                  10                  15
   Glu Ala Gly Ile Arg Tyr Phe Asp Leu Arg Val Ser Ser Lys Pro Gly
               20                  25                  30
   Asp Ala Asp Gln Glu Ile Tyr Phe Ile His Gly Leu Phe Gly Ile Lys
           35                  40                  45
   Val Trp Asp Gly Leu Met Glu Ile Asp Ser Phe Leu Thr Gln His Pro
       50                  55                  60
   Gln Glu Ile Ile Phe Leu Asp Phe Asn His Phe Tyr Ala Met Asp Glu
   65                  70                  75                  80
   Thr His His Lys Cys Leu Val Leu Arg Ile Gln Glu Ala Phe Gly Asn
                   85                  90                  95
   Lys Leu Cys Pro Ala Cys Ser Val Glu Ser Leu Thr Leu Arg Thr Leu
               100                 105                 110
   Trp Glu Lys Asn Cys Gln Val Leu Ile Phe Tyr His Cys Pro Phe Tyr
           115                 120                 125
   Lys Gln Tyr Pro Phe Leu Trp Pro Gly Lys Lys Ile Pro Ala Pro Trp
       130                 135                 140
   Ala Asn Thr Thr Ser Val Arg Lys Leu Ile Leu Phe Leu Glu Thr Thr
   145                 150                 155                 160
   Leu Ser Glu Arg Ala Ser Arg Gly Ser Phe His Val Ser Gln Ala Ile
                   165                 170                 175
   Leu Thr Pro Arg Val Lys Thr Ile Ala Arg Gly Leu Val Gly Gly Leu
               180                 185                 190
   Lys Asn Thr Leu Val His Arg Arg Ser Leu Thr Leu Ser Pro Lys Leu
           195                 200                 205
   Glu Cys Ser Ser Trp Leu Thr Ala Ala Ser Thr Ser Gln Ala Gln Val
       210                 215                 220
   Ile Thr Pro Pro His Arg Gln Gly Phe Thr Met Phe Pro Arg Leu Ile
   225                 230                 235                 240
   Ser Asn Ser Trp Ile Gln Val Ile His Pro Pro Gln Pro Pro Lys Val
                   245                 250                 255
   Pro Gly Leu Gln Ala
               260
<210> 13
   <211> 1426
   <212> DNA
   <213> homo sapiens
<400> 13
   attcgcgccc gtcaggcatg tctgtacact gggtgggcac ctgtcttgtg agtggctccg      60
   ggtgtggctg ctcctcggac tttcagttta tgtaagattt atctctaggg gcctaccttc     120
   ccccatctcc agaggggaac ataagaagtt taacggagct gggactgagc agattaaggg     180
   agtggagcgg aggctgggcc ggagagagtg gggactgtga gtgctagtgg gtaaggatcc     240
   atctgtttgc cccgtccccc agccagaaag gcattttgga aagactggcg tggcaagcgt     300
   cgccctgaaa cgtccacaga gcccaagaag tgatgatcac tgagtgagtg gcactgggct     360
   gagactggcc agtttgttaa caacagggat gctagcagtt aggaaggcca ggaggaaact     420
   caggatgggg accatctgct cccccaaccc cagcgggaca aagacatcat cggaggtctg     480
   caatgccgac tggatggcct cgctcccccc tcacctccac aacctccccc tttccaatct     540
   ggcaatccca ggctcacatg attcattcag ctactgggtg gatgaaaagt ccccagtggg     600
   gcctgaccaa acccaagcta tcaaacgcct cgccaggatc tccttggtga agaagctaat     660
   gaagaagtgg tctgtgactc agaacctgac atttcgagaa cagctggaag ctgggatccg     720
   ctactttgac ctgcgtgtgt cttccaaacc aggggatgcc gaccaggaga tctacttcat     780
   ccatgggctt tttggcatca aggtctggga tgggctgatg gaaattgact cgtttcttac     840
   acagcacccc caggagatta tcttcctgga tttcaaccac ttctatgcca tggatgagac     900
   ccatcacaaa tgcctggttc tgcggatcca ggaggccttt ggaaacaagc tgtgcccagc     960
   ctgcagtgtg gaaagtttga cgctgcgaac tctgtgggag aagaactgcc aggttcttat    1020
   tttctaccac tgtcccttct acaagcagta ccccttcctg tggccaggaa agaagattcc    1080
   agcgccctgg gcaaacacca caagtgtgcg caaactaatc ctcttcttgg agaccactct    1140
   gagtgagcgg gcctcacggg gctccttcca tgtctcccaa gcgatcctca cccccagagt    1200
   gaagaccatt gcccggggct tggttggggg cctcaagaac acgctggttc ataggaatct    1260
   tcctgccatc ctggactggg tgaaaactca gaagcctgga gccatgggtg tcaacatcat    1320
   cacatctgac ttcgtggacc tggtggactt tgctgcgact gtcatcaaag ttgaatgacc    1380
   ttctacagga ggacacaagc tctggcttaa tgctgattta attttt                   1426
<210> 14
   <211> 2289
   <212> DNA
   <213> homo sapiens
<400> 14
   atggcgtccc tgctgcaaga ccagctgacc actgatcagg acttgctgct gatgcaggaa      60
   ggcatgccga tgcgcaaggt gaggtccaaa agctggaaga agctaagata cttcagactt     120
   cagaatgacg gcatgacagt ctggcatgca cggcaggcca ggggcagtgc caagcccagc     180
   ttctcaatct ctgatgtgga gacaatacgt aatggccatg attccgagtt gctgcgtagc     240
   ctggcagagg agctccccct ggagcagggc ttcaccattg tcttccatgg ccgccgctcc     300
   aacctggacc tgatggccaa cagtgttgag gaggcccaga tatggatgcg agggctccag     360
   ctgttggtgg atcttgtcac cagcatggac catcaggagc gcctggacca atggctgagc     420
   gattggtttc aacgtggaga caaaaatcag gatggtaaga tgagtttcca agaagttcag     480
   cggttattgc acctaatgaa tgtggaaatg gaccaagaat atgccttcag tctttttcag     540
   gcagcagaca cgtcccagtc tggaaccctg gaaggagaag aattcgtaca gttctataag     600
   gcattgacta aacgtgctga ggtgcaggaa ctgtttgaaa gtttttcagc tgatgggcag     660
   aagctgactc tgctggaatt tttggatttc ctccaagagg agcagaagga gagagactgc     720
   acctctgagc ttgctctgga actcattgac cgctatgaac cttcagacag tggcaaactg     780
   cggcatgtgc tgagtatgga tggcttcctc agctacctct gctctaagga tggagacatc     840
   ttcaacccag cctgcctccc catctatcag gatatgactc aacccctgaa ccactacttc     900
   atctgctctt ctcataacac ctacctagtg ggggaccagc tttgcggcca gagcagcgtc     960
   gagggatata tacgggccct gaagcggggg tgccgctgcg tggaggtgga tgtatgggat    1020
   ggacctagcg gggaacctgt cgtttaccac ggacacaccc tgacctcccg catcctgttc    1080
   aaagatgtcg tggccacagt agcacagtat gccttccaga catcagacta cccagtcatc    1140
   ttgtccctgg agacccactg cagctgggag cagcagcaga ccatggcccg tcatctgact    1200
   gagatcctgg gggagcagct gctgagcacc accttggatg gggtgctgcc cactcagctg    1260
   ccctcgcctg aggagcttcg gaggaagatc ctggtgaagg ggaagaagtt aacacttgag    1320
   gaagacctgg aatatgagga agaggaagca gaacctgagt tggaagagtc agaattggcg    1380
   ctggagtccc agtttgagac tgagcctgag ccccaggagc agaaccttca gaataaggac    1440
   aaaaagaaga aatccaagcc catcttgtgt ccagccctct cttccctggt tatctacttg    1500
   aagtctgtct cattccgcag cttcacacat tcaaaggagc actaccactt ctacgagata    1560
   tcatctttct ctgaaaccaa ggccaagcgc ctcatcaagg aggctggcaa tgagtttgtg    1620
   cagcacaata cttggcagtt aagccgtgtg tatcccagcg gcctgaggac agactcttcc    1680
   aactacaacc cccaggaact ctggaatgca ggctgccaga tggtggccat gaatatgcag    1740
   actgcagggc ttgaaatgga catctgtgat gggcatttcc gccagaatgg cggctgtggc    1800
   tatgtgctga agccagactt cctgcgtgat atccagagtt ctttccaccc tgagaagccc    1860
   atcagccctt tcaaagccca gactctctta atccaggtga tcagcggtca gcaactcccc    1920
   aaagtggaca agaccaaaga ggggtccatt gtggatccac tggtgaaagt gcagatcttt    1980
   ggcgttcgtc tagacacagc acggcaggag accaactatg tggagaacaa tggttttaat    2040
   ccatactggg ggcagacact atgtttccgg gtgctggtgc ctgaacttgc catgctgcgt    2100
   tttgtggtaa tggattatga ctggaaatcc cgaaatgact ttattggtca gtacaccctg    2160
   ccttggacct gcatgcaaca aggttaccgc cacattcacc tgctgtccaa agatggcatc    2220
   agcctccgcc cagcttccat ctttgtgtat atctgcatcc aggaaggcct ggagggggat    2280
   gagtcctga                                                            2289
<210> 15
   <211> 762
   <212> PRT
   <213> homo sapiens
<400> 15
   Met Ala Ser Leu Leu Gln Asp Gln Leu Thr Thr Asp Gln Asp Leu Leu
   1               5                   10                  15
   Leu Met Gln Glu Gly Met Pro Met Arg Lys Val Arg Ser Lys Ser Trp
               20                  25                  30
   Lys Lys Leu Arg Tyr Phe Arg Leu Gln Asn Asp Gly Met Thr Val Trp
           35                  40                  45
   His Ala Arg Gln Ala Arg Gly Ser Ala Lys Pro Ser Phe Ser Ile Ser
       50                  55                  60
   Asp Val Glu Thr Ile Arg Asn Gly His Asp Ser Glu Leu Leu Arg Ser
   65                  70                  75                  80
   Leu Ala Glu Glu Leu Pro Leu Glu Gln Gly Phe Thr Ile Val Phe His
                   85                  90                  95
   Gly Arg Arg Ser Asn Leu Asp Leu Met Ala Asn Ser Val Glu Glu Ala
               100                 105                 110
   Gln Ile Trp Met Arg Gly Leu Gln Leu Leu Val Asp Leu Val Thr Ser
           115                 120                 125
   Met Asp His Gln Glu Arg Leu Asp Gln Trp Leu Ser Asp Trp Phe Gln
       130                 135                 140
   Arg Gly Asp Lys Asn Gln Asp Gly Lys Met Ser Phe Gln Glu Val Gln
   145                 150                 155                 160
   Arg Leu Leu His Leu Met Asn Val Glu Met Asp Gln Glu Tyr Ala Phe
                   165                 170                 175
   Ser Leu Phe Gln Ala Ala Asp Thr Ser Gln Ser Gly Thr Leu Glu Gly
               180                 185                 190
   Glu Glu Phe Val Gln Phe Tyr Lys Ala Leu Thr Lys Arg Ala Glu Val
           195                 200                 205
   Gln Glu Leu Phe Glu Ser Phe Ser Ala Asp Gly Gln Lys Leu Thr Leu
       210                 215                 220
   Leu Glu Phe Leu Asp Phe Leu Gln Glu Glu Gln Lys Glu Arg Asp Cys
   225                 230                 235                 240
   Thr Ser Glu Leu Ala Leu Glu Leu Ile Asp Arg Tyr Glu Pro Ser Asp
                   245                 250                 255
   Ser Gly Lys Leu Arg His Val Leu Ser Met Asp Gly Phe Leu Ser Tyr
               260                 265                 270
   Leu Cys Ser Lys Asp Gly Asp Ile Phe Asn Pro Ala Cys Leu Pro Ile
           275                 280                 285
   Tyr Gln Asp Met Thr Gln Pro Leu Asn His Tyr Phe Ile Cys Ser Ser
   290 295 300
   His Asn Thr Tyr Leu Val Gly Asp Gln Leu Cys Gly Gln Ser Ser Val
   305 310 315 320
   Glu Gly Tyr Ile Arg Ala Leu Lys Arg Gly Cys Arg Cys Val Glu Val
   325 330 335
   Asp Val Trp Asp Gly Pro Ser Gly Glu Pro Val Val Tyr His Gly His
   340 345 350
   Thr Leu Thr Ser Arg Ile Leu Phe Lys Asp Val Val Ala Thr Val Ala
   355 360 365
   Gln Tyr Ala Phe Gln Thr Ser Asp Tyr Pro Val Ile Leu Ser Leu Glu
   370 375 380
   Thr His Cys Ser Trp Glu Gln Gln Gln Thr Met Ala Arg His Leu Thr
   385 390 395 400
   Glu Ile Leu Gly Glu Gln Leu Leu Ser Thr Thr Leu Asp Gly Val Leu
   405 410 415
   Pro Thr Gln Leu Pro Ser Pro Glu Glu Leu Arg Arg Lys Ile Leu Val
   420 425 430
   Lys Gly Lys Lys Leu Thr Leu Glu Glu Asp Leu Glu Tyr Glu Glu Glu
   435 440 445
   Glu Ala Glu Pro Glu Leu Glu Glu Ser Glu Leu Ala Leu Glu Ser Gln
   450 455 460
   Phe Glu Thr Glu Pro Glu Pro Gln Glu Gln Asn Leu Gln Asn Lys Asp
   465 470 475 480
   Lys Lys Lys Lys Ser Lys Pro Ile Leu Cys Pro Ala Leu Ser Ser Leu
   485 490 495
   Val Ile Tyr Leu Lys Ser Val Ser Phe Arg Ser Phe Thr His Ser Lys
   500 505 510
   Glu His Tyr His Phe Tyr Glu Ile Ser Ser Phe Ser Glu Thr Lys Ala
   515 520 525
   Lys Arg Leu Ile Lys Glu Ala Gly Asn Glu Phe Val Gln His Asn Thr
   530 535 540
   Trp Gln Leu Ser Arg Val Tyr Pro Ser Gly Leu Arg Thr Asp Ser Ser
   545 550 555 560
   Asn Tyr Asn Pro Gln Glu Leu Trp Asn Ala Gly Cys Gln Met Val Ala
   565 570 575
   Met Asn Met Gln Thr Ala Gly Leu Glu Met Asp Ile Cys Asp Gly His
   580 585 590
   Phe Arg Gln Asn Gly Gly Cys Gly Tyr Val Leu Lys Pro Asp Phe Leu
   595 600 605
   Arg Asp Ile Gln Ser Ser Phe His Pro Glu Lys Pro Ile Ser Pro Phe
   610 615 620
   Lys Ala Gln Thr Leu Leu Ile Gln Val Ile Ser Gly Gln Gln Leu Pro
   625 630 635 640
   Lys Val Asp Lys Thr Lys Glu Gly Ser Ile Val Asp Pro Leu Val Lys
   645 650 655
   Val Gln Ile Phe Gly Val Arg Leu Asp Thr Ala Arg Gln Glu Thr Asn
   660 665 670
   Tyr Val Glu Asn Asn Gly Phe Asn Pro Tyr Trp Gly Gln Thr Leu Cys
   675 680 685
   Phe Arg Val Leu Val Pro Glu Leu Ala Met Leu Arg Phe Val Val Met
   690 695 700
   Asp Tyr Asp Trp Lys Ser Arg Asn Asp Phe Ile Gly Gln Tyr Thr Leu
   705 710 715 720
   Pro Trp Thr Cys Met Gln Gln Gly Tyr Arg His Ile His Leu Leu Ser
   725 730 735
   Lys Asp Gly Ile Ser Leu Arg Pro Ala Ser Ile Phe Val Tyr Ile Cys
               740                 745                 750
   Ile Gln Glu Gly Leu Glu Gly Asp Glu Ser
           755                 760
<210> 16
   <211> 210
   <212> DNA
   <213> homo sapiens
<400> 16
   atggcgtccc tgctgcaaga ccagctgacc actgatcagg acttgctgct gatgcaggaa     60
   ggcatgccga tgcgcaagtc tcaatctctg atgtggagac aatacgtaat ggccatgatt    120
   ccgagttgct gcgtagcctg gcagaggagc tccccctgga gcagggcttc accattgtct    180
   tccatggccg ccgctccaac ctggacctga                                     210
<210> 17
   <211> 69
   <212> PRT
   <213> homo sapiens
<400> 17
   Met Ala Ser Leu Leu Gln Asp Gln Leu Thr Thr Asp Gln Asp Leu Leu
    1               5                  10                  15
   Leu Met Gln Glu Gly Met Pro Met Arg Lys Ser Gln Ser Leu Met Trp
               20                  25                  30
   Arg Gln Tyr Val Met Ala Met Ile Pro Ser Cys Cys Val Ala Trp Gln
           35                  40                  45
   Arg Ser Ser Pro Trp Ser Arg Ala Ser Pro Leu Ser Ser Met Ala Ala
       50                  55                  60
   Ala Pro Thr Trp Thr
   65
<210> 18
   <211> 819
   <212> DNA
   <213> homo sapiens
<400> 18
   atggcgtccc tgctgcaaga ccagctgacc actgatcagg acttgctgct gatgcaggaa      60
   ggcatgccga tgcgcaaggt gaggtccaaa agctggaaga agctaagata cttcagactt     120
   cagaatgacg gcatgacagt ctggcatgca cggcaggcca ggggcagtgc caagcccagc     180
   ttctcaatct ctgatgtgga gacaatacgt aatggccatg attccgagtt gctgcgtagc     240
   ctggcagagg agctccccct ggagcagggc ttcaccattg tcttccatgg ccgccgctcc     300
   aacctggacc tgatggccaa cagtgttgag gaggcccaga tatggatgcg agggctccag     360
   ctgttggtgg atcttgtcac cagcatggac catcaggagc gcctggacca atggctgagc     420
   gattggtttc aacgtggaga caaaaatcag gatggtaaga tgagtttcca agaagttcag     480
   cggttattgc acctaatgaa tgtggaaatg gaccaagaat atgccttcag tctttttcag     540
   gcagcagaca cgtcccagtc tggaaccctg gaaggagaag aattcgtaca gttctataag     600
   gcattgacta aacgtgctga ggtgcaggaa ctgtttgaaa gtttttcagc tgatgggcag     660
   aagctgactc tgctggaatt tttggatttc ctccaagagg agcagaagga gagagactgc     720
   acctctgagc ttgctctgga actcattgac cgctatgaac cttcagacag tggagcttcg     780
   gaggaagatc ctggtgaagg ggaagaagtt aacacttga                            819
<210> 19
   <211> 272
   <212> PRT
   <213> homo sapiens
<400> 19
   Met Ala Ser Leu Leu Gln Asp Gln Leu Thr Thr Asp Gln Asp Leu Leu
   1               5                   10                  15
   Leu Met Gln Glu Gly Met Pro Met Arg Lys Val Arg Ser Lys Ser Trp
               20                  25                  30
   Lys Lys Leu Arg Tyr Phe Arg Leu Gln Asn Asp Gly Met Thr Val Trp
           35                  40                  45
   His Ala Arg Gln Ala Arg Gly Ser Ala Lys Pro Ser Phe Ser Ile Ser
       50                  55                  60
   Asp Val Glu Thr Ile Arg Asn Gly His Asp Ser Glu Leu Leu Arg Ser
   65                  70                  75                  80
   Leu Ala Glu Glu Leu Pro Leu Glu Gln Gly Phe Thr Ile Val Phe His
                   85                  90                  95
   Gly Arg Arg Ser Asn Leu Asp Leu Met Ala Asn Ser Val Glu Glu Ala
               100                 105                 110
   Gln Ile Trp Met Arg Gly Leu Gln Leu Leu Val Asp Leu Val Thr Ser
           115                 120                 125
   Met Asp His Gln Glu Arg Leu Asp Gln Trp Leu Ser Asp Trp Phe Gln
       130                 135                 140
   Arg Gly Asp Lys Asn Gln Asp Gly Lys Met Ser Phe Gln Glu Val Gln
   145                 150                 155                 160
   Arg Leu Leu His Leu Met Asn Val Glu Met Asp Gln Glu Tyr Ala Phe
                   165                 170                 175
   Ser Leu Phe Gln Ala Ala Asp Thr Ser Gln Ser Gly Thr Leu Glu Gly
               180                 185                 190
   Glu Glu Phe Val Gln Phe Tyr Lys Ala Leu Thr Lys Arg Ala Glu Val
           195                 200                 205
   Gln Glu Leu Phe Glu Ser Phe Ser Ala Asp Gly Gln Lys Leu Thr Leu
       210                 215                 220
   Leu Glu Phe Leu Asp Phe Leu Gln Glu Glu Gln Lys Glu Arg Asp Cys
   225                 230                 235                 240
   Thr Ser Glu Leu Ala Leu Glu Leu Ile Asp Arg Tyr Glu Pro Ser Asp
                   245                 250                 255
   Ser Gly Ala Ser Glu Glu Asp Pro Gly Glu Gly Glu Glu Val Asn Thr
               260                 265                 270
<210> 20
   <211> 2709
   <212> DNA
   <213> homo sapiens
<400> 20
   aagagctcac acctttcccc ttcttactgc ttccctccgg ctataacttg ccagtcacag      60
   cagccagctg ctgtagaaga ggggaggaaa caagccagtg caaggggagc aaaagagaaa     120
   aggagccagg ctgggcttcc tgatcccaca gcatcgcaga gctcgggagg cacagctcac     180
   agacacagga aacacaggac tgctattctg ctctcctgcc cacggtgatc tggtgccagc     240
   tggtggaaca gtgggtgatg gcgtccctgc tgcaagacca gctgaccact gatcaggact     300
   tgctgctgat gcaggaaggc atgccgatgc gcaaggtgag gtccaaaagc tggaagaagc     360
   taagatactt cagacttcag aatgacggca tgacagtctg gcatgcacgg caggccaggg     420
   gcagtgccaa gcccagcttc tcaatctctg atgtggagac aatacgtaat ggccatgatt     480
   ccgagttgct gcgtagcctg gcagaggagc tccccctgga gcagggcttc accattgtct     540
   tccatggccg ccgctccaac ctggacctga tggccaacag tgttgaggag gcccagatat     600
   ggatgcgagg gctccagctg ttggtggatc ttgtcaccag catggaccat caggagcgcc     660
   tggaccaatg gctgagcgat tggtttcaac gtggagacaa aaatcaggat ggtaagatga      720
   gtttccaaga agttcagcgg ttattgcacc taatgaatgt ggaaatggac caagaatatg      780
   ccttcagtct ttttcaggca gcagacacgt cccagtctgg aaccctggaa ggagaagaat       840
   tcgtacagtt ctataaggca ttgactaaac gtgctgaggt gcaggaactg tttgaaagtt      900
   tttcagctga tgggcagaag ctgactctgc tggaattttt ggatttcctc caagaggagc      960
   agaaggagag agactgcacc tctgagcttg ctctggaact cattgaccgc tatgaacctt     1020
   cagacagtgg caaactgcgg catgtgctga gtatggatgg cttcctcagc tacctctgct     1080
   ctaaggatgg agacatcttc aacccagcct gcctccccat ctatcaggat atgactcaac     1140
   ccctgaacca ctacttcatc tgctcttctc ataacaccta cctagtgggg gaccagcttt     1200
   gcggccagag cagcgtcgag ggatatatac gggccctgaa gcgggggtgc cgctgcgtgg     1260
   aggtggatgt atgggatgga cctagcgggg aacctgtcgt ttaccacgga cacaccctga     1320
   cctcccgcat cctgttcaaa gatgtcgtgg ccacagtagc acagtatgcc ttccagacat     1380
   cagactaccc agtcatcttg tccctggaga cccactgcag ctgggagcag cagcagacca     1440
   tggcccgtca tctgactgag atcctggggg agcagctgct gagcaccacc ttggatgggg     1500
   tgctgcccac tcagctgccc tcgcctgagg agcttcggag gaagatcctg gtgaagggga     1560
   agaagttaac acttgaggaa gacctggaat atgaggaaga ggaagcagaa cctgagttgg     1620
   aagagtcaga attggcgctg gagtcccagt ttgagactga gcctgagccc caggagcaga     1680
   accttcagaa taaggacaaa aagaagaaat ccaagcccat cttgtgtcca gccctctctt     1740
   ccctggttat ctacttgaag tctgtctcat tccgcagctt cacacattca aaggagcact     1800
   accacttcta cgagatatca tctttctctg aaaccaaggc caagcgcctc atcaaggagg     1860
   ctggcaatga gtttgtgcag cacaatactt ggcagttaag ccgtgtgtat cccagcggcc     1920
   tgaggacaga ctcttccaac tacaaccccc aggaactctg gaatgcaggc tgccagatgg     1980
   tggccatgaa tatgcagact gcagggcttg aaatggacat ctgtgatggg catttccgcc     2040
   agaatggcgg ctgtggctat gtgctgaagc cagacttcct gcgtgatatc cagagttctt     2100
   tccaccctga gaagcccatc agccctttca aagcccagac tctcttaatc caggtgatca     2160
   gcggtcagca actccccaaa gtggacaaga ccaaagaggg gtccattgtg gatccactgg     2220
   tgaaagtgca gatctttggc gttcgtctag acacagcacg gcaggagacc aactatgtgg     2280
   agaacaatgg ttttaatcca tactgggggc agacactatg tttccgggtg ctggtgcctg     2340
   aacttgccat gctgcgtttt gtggtaatgg attatgactg gaaatcccga aatgacttta     2400
   ttggtcagta caccctgcct tggacctgca tgcaacaagg ttaccgccac attcacctgc     2460
   tgtccaaaga tggcatcagc ctccgcccag cttccatctt tgtgtatatc tgcatccagg     2520
   aaggcctgga gggggatgag tcctgaggtg ggcatttcac gggaagggtt ggtatgctgg     2580
   ctttagacgg ggagaaacat ctggaaggat gctcgagaga acaaatggag gtggtgaaaa     2640
   tcaagctttg gattgtgcat tcctaggcac aaaattacct cattcttcct aacaagcaat     2700
   ctgggacct                                                             2709

## Description

### 1. INTRODUCTION

The present invention relates to the discovery, identification, and characterization of novel human polynucleotides encoding proteins sharing sequence similarity with mammalian phospholipases. The invention encompasses the described polynucleotides, host cell expression systems, the encoded proteins, fusion proteins, polypeptides and peptides, antibodies to the encoded proteins and peptides, and genetically engineered animals that either lack or over express the disclosed polynucleotides, antagonists and agonists of the proteins, and other compounds that modulate the expression or activity of the proteins encoded by the disclosed polynucleotides that can be used for diagnosis, drug screening, clinical trial monitoring and the treatment of diseases and disorders.

### 2. BACKGROUND OF THE INVENTION

Phospholipases hydrolyze phospholipids and can play a key role in the cell activation and signal transduction. As such, phospholipases have been associated with, *inter alia*, development, inflammation, infectious disease, and cancer.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with animal phospholipases, including phospholipase C delta-4.

The novel human nucleic acid (cDNA) sequences described herein encode proteins/open reading frames (ORFs) of 239, 329, 351, 149, 239, 261, 762, 69, and 272 amino acids in length (see SEQ ID NOS: 2, 4, 6, 8, 10, 12, 15, 17 and 19 respectively).

The invention also encompasses agonists and antagonists of the described NHPs, including small molecules, large molecules, mutant NHPs, or portions thereof, that compete with native NHP, peptides, and antibodies, as well as nucleotide sequences that can be used to inhibit the expression of the described NHPs (*e.g*., antisense and ribozyme molecules, and gene or regulatory sequence replacement constructs) or to enhance the expression of the described NHP polynucleotides (*e.g.*, expression constructs that place the described polynucleotide under the control of a strong promoter system), and transgenic animals that express a NHP transgene, or "knock-outs" (which can be conditional) that do not express a functional NHP. Knock-out mice can be produced in several ways, one of which involves the use of mouse embryonic stem cells ("ES cells") lines that contain gene trap mutations in a murine homolog of at least one of the described NHPs. When the unique NHP sequences described in SEQ ID NOS:1-20 are "knocked-out" they provide a method of identifying phenotypic expression of the particular gene as well as a method of assigning function to previously unknown genes. Additionally, the unique NHP sequences described in SEQ ID NOS:1-20 are useful for the identification of coding sequence and the mapping a unique gene to a particular chromosome.

Further, the present invention also relates to processes for identifying compounds that modulate, *i.e*., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of the described NHP ORFs encoding the described NHP amino acid sequences. SEQ ID NO:13 and 20 describe nucleotides encoding a NHP ORF with regions of flanking sequence.

### 5. DETAILED DESCRIPTION OF THE INVENTION

The NHPs, described for the first time herein in SEQ ID NOS: 1- 13, are novel proteins that are clearly expressed in, *inter alia,* human cell lines, human fetal brain, brain, cerebellum, spinal cord, thymus, spleen, testis, thyroid, adrenal gland, small intestine, colon, placenta, adipose, rectum, and gene trapped cells. The described sequences were compiled from gene trapped cDNAs, human genomic sequence, and clones isolated from a human fetal brain cDNA librarys (Edge Biosystems, Gaithersburg, MD). The NHPs, described for the first time herein in SEQ ID NOS: 14-20, are novel proteins that are clearly expressed in, *inter alia,* human cell lines, human fetal brain, brain, testis, skeletal muscle, pericardium, trachea, and gene trapped cells. The described sequences were compiled from gene trapped cDNAs, human genomic sequence, and clones isolated from a human trachea cDNA library (Edge Biosystems, Gaithersburg, MD).

The present invention encompasses the nucleotides presented in the Sequence Listing, host cells expressing such nucleotides, the expression products of such nucleotides, and: (a) nucleotides that encode mammalian homologs of the described polynucleotides, including the specifically described NHPs, and the NHP products; (b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal (or hydrophobic transmembrane) sequence is deleted; (d) nucleotides that encode chimeric fusion proteins containing all or a portion of a coding region of an NHP, or one of its domains (*e.g*., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing. As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, *e.g.*, hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F. M. *et al.,* eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of a DNA sequence that encodes and expresses an amino acid sequence presented in the Sequence Listing under moderately stringent conditions, *e.g.*, washing in 0.2xSSC/0.1% SDS at 42°C (Ausubel et al., 1989, *supra*), yet still encodes a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding NHP ORFs, or their functional equivalents, encoded by polynucleotide sequences that are about 99, 95, 90, or about 85 percent similar or identical to corresponding regions of the nucleotide sequences of the Sequence Listing (as measured by BLAST sequence comparison analysis using, for example, the GCG sequence analysis package using standard default settings).

The invention also includes nucleic acid molecules, preferably DNA molecules, that hybridize to, and are therefore the complements of, the described NHP nucleotide sequences. Such hybridization conditions can be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-20 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (*i.e.,* gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-20, or an amino acid sequence encoded thereby. Methods for attaching biopolymers to, or synthesizing biopolymers on, solid support matrices, and conducting binding studies thereon are disclosed in, *inter alia,* U.S. Patent Nos. 5,700,637, 5,556,752, 5,744,305, 4,631,211, 5,445,934, 5,252,743, 4,713,326, 5,424,186, and 4,689,405.

Addressable arrays comprising sequences first disclosed in SEQ ID NOS:1-20 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides, and more preferably 25 nucleotides from the sequences first disclosed in SEQ ID NOS:1-20.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence can be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-20 provides detailed information about transcriptional changes involved in a specific pathway, potentially leading to the identification of novel components or gene functions that manifest themselves as novel phenotypes.

Probes consisting of sequences first disclosed in SEQ ID NOS:1-20 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-20 can be utilized in microarrays or other assay formats, to screen collections of genetic material from patients who have a particular medical condition. These investigations can also be carried out using the sequences first disclosed in SEQ ID NOS:1-20 *in silico* and by comparing previously collected genetic databases and the disclosed sequences using computer software known to those in the art.

Thus, the sequences first disclosed in SEQ ID NOS:1-20 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-20. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (*e.g*., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relatve to one or more additional sequence(s) or one or more restriction sites present in the disclosed sequence.

For oligonucleotide probes, highly stringent conditions may refer, *e.g.*, to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. 15:6625-6641). The oligonucleotide is a 2'-O-methylribonucleotide (Inoue *et al.*, 1987, Nucl. Acids Res. *15*:6131-6148), or a chimeric RNA-DNA analogue (Inoue *et al*., 1987, FEBS Lett. *215*:327-330). Alternatively, double stranded RNA can be used to disrupt the expression and function of a targeted NHP.

Oligonucleotides of the invention can be synthesized by standard methods known in the art, *e.g.* by use of an automated DNA synthesizer (such as are commercially available from Biosearch, Applied Biosystems, etc.). As examples, phosphorothioate oligonucleotides can be synthesized by the method of Stein *et al*. (1988, Nucl. Acids Res. 16:3209), and methylphosphonate oligonucleotides can be prepared by use of controlled pore glass polymer supports (Sarin *et al.*, 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al.,* 1989, Molecular Cloning, A Laboratory Manual (and periodic updates thereof), Cold Springs Harbor Press, N.Y.; and Ausubel *et al.,* 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g*., splice acceptor and/or donor sites), etc., that can be used in diagnostics and pharmacogenomics.

Further, a NHP gene homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e.*, one known, or suspected, to express a NHP gene). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g.*, Sambrook *et al.,* 1989, *supra.*

A cDNA encoding a mutant NHP gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of, or known to carry a mutant NHP allele (*e.g*., a person manifesting a NHP-associated phenotype such as, for example, obesity, high blood pressure, connective tissue disorders, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of, or known to carry, such a mutant allele. In this manner, gene products made by the putatively mutant tissue can be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor, NY).

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e.g.*, as a result of a missense or a frameshift mutation), polyclonal antibodies to a NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.*, antisense); (b) DNA expression vectors that contain any of the foregoing NHP coding sequences operatively associated with a regulatory element that directs the expression of the coding sequences (for example, baculo virus as described in U.S. Patent No. 5,869,336; (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i.e.*, gene activation). As used herein, regulatory elements include, but are not limited to, inducible and non-inducible promoters, enhancers, operators and other elements known to those skilled in the art that drive and regulate expression. Such regulatory elements include but are not limited to, the cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of a NHP, as well as compounds or nucleotide constructs that inhibit expression of a NHP gene (transcription factor inhibitors, antisense and ribozyme molecules, or gene or regulatory sequence replacement constructs), or promote the expression of a NHP (*e.g.*, expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for an NHP, but can also identify compounds that trigger NHP-mediated activities or pathways.

Finally, the NHP products can be used as therapeutics. For example, soluble derivatives such as NHP peptides/domains corresponding to NHPs, NHP fusion protein products (especially NHP-Ig fusion proteins, *i.e.*, fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP receptor. Nucleotide constructs encoding such NHP products can be used to genetically engineer host cells to express such products *in vivo;* these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression.

Various aspects of the invention are described in greater detail in the subsections below.

### 5.1 THE NHP SEQUENCES

The cDNA sequences and the corresponding deduced amino acid sequences of the described NHPs are presented in the Sequence Listing. SEQ ID NOS:13 and 20 describe NHP ORFs as well as flanking regions. The NHP nucleotides were obtained from human cDNA libraries using probes and/or primers generated from human gene trapped sequence tags. Expression analysis has provided evidence that some of the described NHPs are widely expressed (SEQ ID NOS:1-13) and some of the described NHPs (SEQ ID NOS:14-20) have a fairly restricted pattern of expression including those that share structural similarity with phospholipase C delta-4 . Given the importance of phospholipases, similar molecules and activities have been subject to considerable scientific scrutiny as demonstrated in U.S. Patents Nos. 5,859,222 and 5,587,306, which describe molecules encoding phospholipase activities that are similar to those of the disclosed NHPs as well as a variety of uses and applications for which the described NHPs can be applied.

### 5.2 NHPS AND NHP POLYPEPTIDES

NHPs, polypeptides, peptide fragments, mutated, truncated, or deleted forms of the NHPs, and/or NHP fusion proteins can be prepared for a variety of uses. These uses include but are not limited to the generation of antibodies, as reagents in diagnostic assays, the identification of other cellular gene products related to a NHP, as reagents in assays for screening for compounds that can be as pharmaceutical reagents useful in the therapeutic treatment of mental, biological, or medical disorders and diseases. Given the similarity information and expression data, the described NHPs can be targeted (by drugs, oligos, antibodies, etc,) in order to treat disease, or to therapeutically augment the efficacy of, for example, chemotherapeutic agents used in the treatment of breast or prostate cancer.

The Sequence Listing discloses the amino acid sequences encoded by the described NHP polynucleotides. The NHPs typically display have initiator methionines in DNA sequence contexts consistent with a translation initiation site.

The NHP amino acid sequences of the invention include the amino acid sequence presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al,* eds., Scientific American Books, New York, NY) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e.g*., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, the NHP peptide or polypeptide is thought to be membrane protein, the hydrophobic regions of the protein can be excised and the resulting soluble peptide or polypeptide can be recovered from the culture media. Such expression systems also encompass engineered host cells that express a NHP, or functional equivalent, *in situ*. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e.g.*, in drug screening assays.

The expression systems that may be used for purposes of the invention include but are not limited to microorganisms such as bacteria (*e.g*., *E. coli, B. subtilis*) transformed with recombinant bacteriophage DNA, plasmid DNA or cosmid DNA expression vectors containing NHP nucleotide sequences; yeast (*e.g., Saccharomyces, Pichia*) transformed with recombinant yeast expression vectors containing NHP nucleotide sequences; insect cell systems infected with recombinant virus expression vectors (*e.g*., baculovirus) containing NHP sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing NHP nucleotide sequences; or mammalian cell systems (*e.g*., COS, CHO, BHK, 293, 3T3) harboring recombinant expression constructs containing promoters derived from the genome of mammalian cells (*e.g.*, metallothionein promoter) or from mammalian viruses (*e.g.*, the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E. coli* expression vector pUR278 (Ruther *et al.*, 1983, EMBO J. *2*:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109; Van Heeke & Schuster, 1989, J. Biol. Chem. *264*:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP coding sequence may be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e*., virus lacking the proteinaceous coat coded for by the polyhedrin gene). These recombinant viruses are then used to infect Spodoptera frugiperda cells in which the inserted sequence is expressed (*e.g.*, see Smith *et al.,* 1983, J. Virol. *46*:584; U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing a NHP product in infected hosts (*e.g.*, See Logan & Shenk, 1984, Proc. Natl. Acad. Sci. USA *81:*3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter *et al.*, 1987, Methods in Enzymol. *153*:516-544).

In addition, a host cell strain may be chosen that modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g.*, promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, *et al*., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al.*, 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA 77:3567; O' Hare, *et al.*, 1981, Proc. Natl. Acad. Sci. USA *78*:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, *et al*., 1981, J. Mol. Biol. *150*:1); and hygro, which confers resistance to hygromycin (Santerre, *et al.,* 1984, Gene *30*:147).

Alternatively, any fusion protein can be readily purified by utilizing an antibody specific for the fusion protein being expressed. For example, a system described by Janknecht *et al*. allows for the ready purification of non-denatured fusion proteins expressed in human cell lines (Janknecht, *et al.,* 1991, Proc. Natl. Acad. Sci. USA *88*:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in Liposomes:A Practical Approach, New, RRC ed., Oxford University Press, New York and in U.S. Patents Nos. 4,594,595, 5,459,127, 5,948,767 and 6,110,490. Additionally embodied are novel protein constructs engineered in such a way that they facilitate transport of the NHP to the target site or desired organ. This goal may be achieved by coupling of the NHP to a cytokine or other ligand that provides targeting specificity, and/or to a protein transducing domain (see generally U.S. applications Ser. No. 60/111,701 and 60/056,713, for examples of such transducing sequences) to facilitate passage across cellular membranes if needed and can optionally be engineered to include nuclear localization sequences when desired.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP gene product. Additionally, such antibodies can be used in conjunction with gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with a NHP, an NHP peptide (*e.g*., one corresponding to a functional domain of an NHP), truncated NHP polypeptides (NHP in which one or more domains have been deleted), functional equivalents of the NHP, or mutated variant of the NHP. Such host animals may include, but are not limited to, pigs, rabbits, mice, goats, and rats, to name but a few. Various adjuvants may be used to increase the immunological response, depending on the host species, including but not limited to, Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature *256*:495-497; and U.S. Patent No. 4,376,110), the human B-cell hybridoma technique (Kosbor et *al*., 1983, Immunology Today *4*:72; Cole *et al.*, 1983, Proc. Natl. Acad. Sci. USA *80*:2026-2030), and the EBV-hybridoma technique (1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison *et al.,* 1984, Proc. Natl. Acad. Sci., *81:*6851-6855; Neuberger *et al.,* 1984, Nature, *312*:604-608; Takeda *et al*., 1985, Nature, *314*:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397. Also encompassed by the present invention is the use of fully humanized monoclonal antibodies as described in US Patent No. 6,150,584.

Alternatively, techniques described for the production of single chain antibodies (U.S. Patent 4,946,778; Bird, 1988, Science *242*:423-426; Huston *et al.,* 1988, Proc. Natl. Acad. Sci. USA *85*:5879-5883; and Ward *et al.,* 1989, Nature *341:*544-546) can be adapted to produce single chain antibodies against NHP gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which may be produced by pepsin digestion of the antibody molecule and the Fab fragments which may be generated by reducing the disulfide bridges of the F(ab')₂ fragments. Alternatively, Fab expression libraries may be constructed (Huse *et al.,* 1989, Science, *246*:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, *e.g.,* Greenspan & Bona, 1993, FASEB J *7(5)*:437-444; and Nissinoff, 1991, J. Immunol. *147(8):*2429-2438). For example antibodies which bind to a NHP domain and competitively inhibit the binding of NHP to its cognate receptor can be used to generate anti-idiotypes that "mimic" the NHP and, therefore, bind and activate or neutralize a receptor. Such anti-idiotypic antibodies or Fab fragments of such anti-idiotypes can be used in therapeutic regimens involving a NHP mediated pathway.

## Claims

1. An isolated nucleic acid molecule comprising a nucleotide sequence that:
(a) encodes the amino acid sequence shown in SEQ ID NO: 15; and
(b) hybridizes under stringent conditions to the nucleotide sequence of SEQ ID NO: 14 or the complement thereof.

2. An isolated nucleic acid molecule encoding the amino acid sequence described in SEQ ID NO: 15.

3. An isolated polypeptide comprising the amino acid sequence disclosed in SEQ ID NO: 15.

4. An isolated nucleic acid molecule encoding the amino acid sequence described in SEQ ID NO: 17.

5. An isolated polypeptide comprising the amino acid sequence disclosed in SEQ ID NO: 17.

6. An isolated nucleic acid molecule encoding the amino acid sequence described in SEQ ID NO: 19.

7. An isolated polypeptide comprising the amino acid sequence disclosed in SEQ ID NO: 19.

## Patentansprüche

1. Ein isoliertes Nukleinsäuremolekül umfassend eine Nukleotidsequenz, die:
(a) für die in SEQ ID NO: 15 gezeigte Aminosäuresequenz kodiert; und
(b) unter stringenten Bedingungen an die Nukleotidsequenz aus SEQ ID NO: 14 oder das Komplement davon hybridisiert.

2. Ein isoliertes Nukleinsäuremolekül, das für die Aminosäuresequenz, die in SEQ ID NO: 15 beschrieben ist, kodiert.

3. Ein isoliertes Polypeptid umfassend die Aminosäuresequenz, die in SEQ ID NO: 15 offenbart ist.

4. Ein isoliertes Nukleinsäuremolekül, das für die Aminosäuresequenz, die in SEQ ID NO: 17 beschrieben ist, kodiert.

5. Ein isoliertes Polypeptid, das die Aminosäuresequenz, die in SEQ ID NO: 17 offenbart ist, umfasst.

6. Ein isoliertes Nukleinsäuremolekül, das für die in SEQ ID NO: 19 beschriebene Aminosäuresequenz kodiert.

7. Ein isoliertes Polypeptid, das die Aminosäuresequenz, die in SEQ ID NO: 19 offenbart ist, umfasst.

## Revendications

1. Molécule isolée d'acide nucléique comprenant une séquence nucléotidique qui :
(a) code pour la séquence d'acides aminés représentée dans SEQ ID NO : 15 ; et
(b) s'hybride dans des conditions stringentes à la séquence nucléotidique de SEQ ID NO : 14 ou au complémentaire de celle-ci

2. Molécule isolée d'acide nucléique codant pour la séquence d'acides aminés décrite dans SEQ ID NO : 15.

3. Polypeptide isolé comprenant la séquence d'acides aminés décrite dans SEQ ID NO : 15.

4. Molécule isolée d'acide nucléique codant pour la séquence d'acides aminés décrite dans SEQ ID NO : 17.

5. Polypeptide isolé comprenant la séquence d'acides aminés décrite dans SEQ ID NO : 17.

6. Molécule isolée d'acide nucléique codant pour la séquence d'acides aminés décrite dans SEQ ID NO : 19.

7. Polypeptide isolé comprenant la séquence d'acides aminés décrite dans SEQ ID NO : 19.
